# EUROPEAN PATENT APPLICATION

(11) **EP 2 540 212 A1**
(43) Date of publication of application: **02.01.2013**
(21) Application number: 12173723.3
(22) Date of filing: 27.06.2012
(51) Int. Cl.: A61B 1/005, A61B 1/00

(54) **Remote accelerometer for articulation of a video probe**

(30) Priority: 30.06.2011 US 201113173280
(71) Applicant: General Electric Company, Schenectady, NY 12345 (US)
(72) Inventor: McClung, Daniel Haddon John, Jamesville, NY New York 13078 (US)
(74) Representative: Picker, Madeline Margaret

(57) **Abstract**

An articulated video probe system (10) and associated method. The system includes an articulated video probe (26) for in-taking and transmitting an image of an area. The articulated video probe (26) is moveable for changing the in-taken image. The system includes a unit for causing the video probe (26) to move. The unit utilizing a received movement command signal to move the articulated video probe (26). The system includes an accelerometer (58) configured to sense motion and transmit the movement command signal to the unit for causing the video probe (26) to move.

## Description

### BACKGROUND OF THE INVENTION

The invention relates to a probe articulation system for moving a video probe and, more particularly, to a probe articulation system that includes an accelerometer remote.

A video probe can be used to capture video and/or images within an area. The video probe can include a camera attached to a probe. The camera and a portion of the probe can be inserted into the area to capture and deliver video and images. Once inserted, the video probe can be articulated in a 360° range of motion to capture video and images in a variety of locations within the area. Movement of the video probe can be controlled by an operator control input unit. The operator control input unit can include a joystick and/or buttons to simultaneously move the video probe and also take pictures and/or video of the area. However, a joystick has a relatively small range of motion, such that a small translation of the joystick can result in a large articulation of the video probe. A large articulation of the video probe may be unfavorable if the user only wants to articulate the video probe a small distance. Additionally, a joystick may lead to inconsistent, uneven, and/or bumpy articulation of the video probe. Thus, a method and device of increasing the range of motion of the operator control input unit to enhance control of the video probe would be beneficial.

### BRIEF DESCRIPTION OF THE INVENTION

The following summary presents a simplified summary in order to provide a basic understanding of some aspects of the systems and/or methods discussed herein. This summary is not an extensive overview of the systems and/or methods discussed herein. It is not intended to identify key/critical elements or to delineate the scope of such systems and/or methods. Its sole purpose is to present some concepts in a simplified form as a prelude to the more detailed description that is presented later.

In accordance with one aspect, the present invention provides an articulated video probe system. The system includes an articulated video probe for in-taking and transmitting an image of an area. The articulated video probe is moveable for changing the in-taken image. The system includes a unit for causing the video probe to move. The unit utilizing a received movement command signal to move the articulated video probe. The system includes an accelerometer configured to sense motion and transmit the movement command signal to the unit for causing the video probe to move.

In accordance with another aspect, the present invention provides a method of operating a video probe system which has an articulated video probe for in-taking and transmitting an image of an area. The method includes sensing motion via an accelerometer and transmitting a movement command signal indicative of the sensed motion. The method includes utilizing the movement command signal at a unit to cause the video probe to move. The method includes moving the articulated video probe for changing the in-taken image.

### BRIEF DESCRIPTION OF THE DRAWINGS

The foregoing and other aspects of the invention will become apparent to those skilled in the art to which the invention relates upon reading the following description with reference to the accompanying drawings, in which:
Fig. 1 is an illustration of an example articulated video probe system in accordance with an aspect of the present invention;
Fig. 2 is a block diagram of an example operator control input unit of the articulated video probe system shown within Fig. 1; and
Fig. 3 is a block diagram of another example operator control input unit, which is freestanding from a control/display unit of an articulated video probe system but is integrated to and/or in a shared arrangement with at least one of a variety of other components/devices.

### DETAILED DESCRIPTION OF EMBODIMENTS OF THE INVENTION

Example embodiments that incorporate one or more aspects of the invention are described and illustrated in the drawings. These illustrated examples are not intended to be a limitation on the invention. For example, one or more aspects of the invention can be utilized in other embodiments and even other types of devices. Moreover, certain terminology is used herein for convenience only and is not to be taken as a limitation on the invention. Still further, in the drawings, the same reference numerals are employed for designating the same elements.

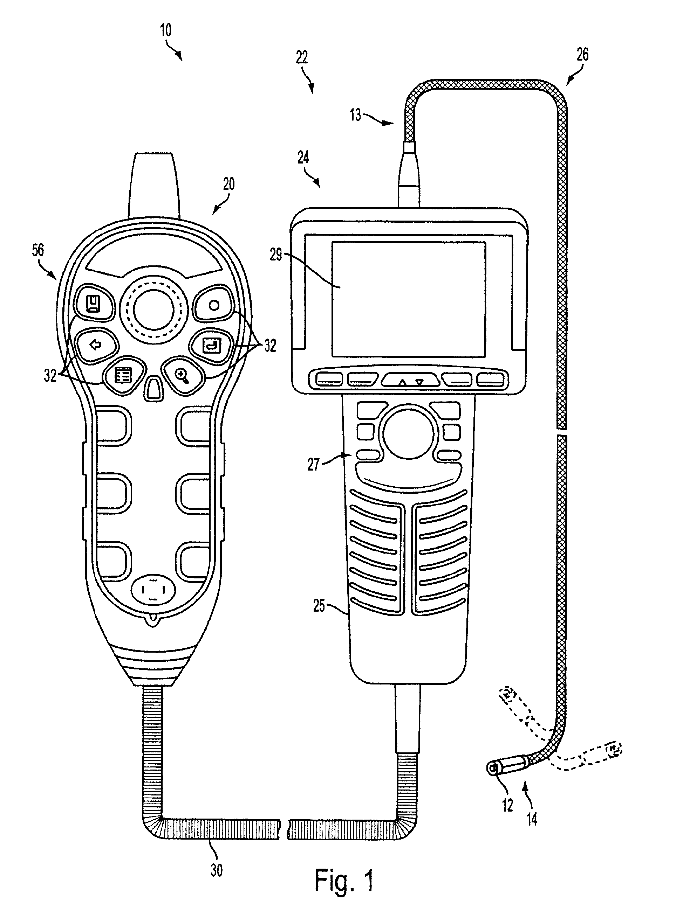

Fig. 1 illustrates an example articulated video probe system 10 in accordance with an aspect of the invention. The shown example articulated video probe system 10 includes an operator control input unit 20, a probe articulation control/display unit 22 and an articulated video probe 26.

The articulated video probe 26 can be of known construction and configuration. The probe 26 can include an elongated, flexible structure, in which a portion of the probe 26 can be moved and/or articulated (as shown by the phantom drawn positions within Fig. 1). The probe 26 shown and described herein is one example of a variety of different embodiments of the probe 26. It is to be understood that in further examples, the probe 26 could be longer, shorter, thicker, thinner, etc. The probe 26 can include an elongated tube having a substantially hollow center portion. The hollow center portion can extend partially or completely along the entire length of the probe 26. The hollow center portion of the probe 26 can be sized to receive wires, cables, fiber optic bundles, or the like. Accordingly, the probe 26 can simultaneously house the wires, articulation cables, fiber optic bundles, etc. while also providing protection to the wires from scratches, tears, or the like.

In general, the articulated video probe 26 is elongate so as to permit insertion of the video probe into areas that have some level of limitation concerning physical/visual accessibility. The length of the elongation of the video probe 26 can be varied as desired (Fig. 1 shows a tear line to convey the concept of unspecified length). The video probe 26 has a camera 12 or similar image in-taking device located at a distal end 14 of the probe. When the video probe 26, with the camera 12 located thereon, is inserted into an area (e.g., a limited access area of a structure, device, etc.), the camera can be operated to in-taken images of the area.

The articulated video probe 26 can be moved, such as by bending, rotating, or the like. Specifically, the distal end 14, with the camera 12 located thereat, can move, bend, rotate, etc. such that the camera is pointed/directed in different directions/orientations so that the image being in-taken by the camera 12 can be varied. In sum, different portions of the area can be imaged by the camera 12 via articulation of the video probe 26. The pointing/directing can be accomplished via various mechanisms such as via application of force to control wires extending along and within the video probe 26 from the control/display unit 22 to the distal end of the video probe 26. Forces can be applied to the control wires via servomotors or the like within the control/display unit 22. It is contemplated that the distal end 14 can be manipulated/moved to be able to capture images in a substantially 360° range of motion.

It is to be appreciated that the control/display unit 22 may have a level of ergonomic design so that the unit can be easily handheld. The shown example of the control/display unit 22 includes a handle portion 25. The handle portion 25 can be sized and shaped to be grasped by a human hand. The control/display unit 22, e.g., at or near the handle portion 25, can include one or more control/function buttons 27 that allow a user to control and/or input information to the control/display unit 22. The control/function associated with each button may be varied and need not be a specific limitation upon the present invention. It is to be understood that the handle portion 25 is not limited to structure in the shown example, and can take on a number of configurations and structures. In the shown example, the handle portion 25 is shown to be substantially rectangular, however, in a further example, the handle portion 25 could include any of a variety of shapes. Similarly, the handle portion 25 could include more or fewer buttons, and, in a further example, could include a trigger switch, joystick, or the like.

The control/display unit 22 further includes a display apparatus 24. Within the shown example, the display apparatus 24 is located above the handle portion 25. The display apparatus 24 includes a screen 29 and associated video controllers, drivers, etc. to provide imagery upon the screen 29. The screen 29 can display images in black and white or in color, and can take on a number of sizes and shapes. The screen 29, via the video controllers, drivers, etc., is in communication with the camera 12 through communication wires in the probe 26. Accordingly, the display apparatus 24 can receive image-conveying signals transmitted from the camera 12 and can display the images/video from the camera 12 on the screen 29. A user can watch the screen 29 to see live images from the camera 12. Also, it is contemplated that the control/display unit 22 may include structure to record video and/or deliver video to an external recording and/or viewing arrangement. The shown example display apparatus 24 also includes one or more buttons, input devices, or the like. The control/function associated with each button, etc. may be varied and need not be a specific limitation upon the present invention.

Within the shown example, the operator control input unit 20 is a separate unit from the control/display unit 22. However, as also shown within the example, the operator control input unit 20 is operatively connected to the control/display unit 22 via a transmission line 30. The length of the transmission line 30 can be varied as desired (Fig. 1 shows a tear line to convey the concept of unspecified length). The operator control input unit 20 is a user input device so that the user can input movement control input to the control/display unit 22 to cause the movement of the video probe 26. Specifically, the user can hold and/or manipulate the operator control input unit 20 to control movement of the distal end 14 of the probe 26. As such, the operator control input unit 20 can be considered to be the "remote" that controls the movement. Also along these lines the operator control unit 20 can be ergonomic designed so that the unit can be easily handheld.

The operator control input unit 20 commands to the control/display unit 22 could include, but are not limited to, commands for movement of the distal end 14 of the probe 26. The operator control input unit 20 can include a number of structures that can provide control input. In the shown example, the operator control input unit 20 includes a keypad 56 having inputs 32. The inputs 32 could include one or more inputs, and can be pressed to allow a user to input commands. The inputs 32 are not limited to the shown example, and can include more or fewer inputs, if necessary. Similarly, the inputs 32 are shown to include buttons, however, other inputs are contemplated, such as a joystick, trigger button, switch, keys, etc. Similarly, the inputs 32 can be provided on a number of different locations and places throughout the operator control input unit 20, and are not limited to the shown example.

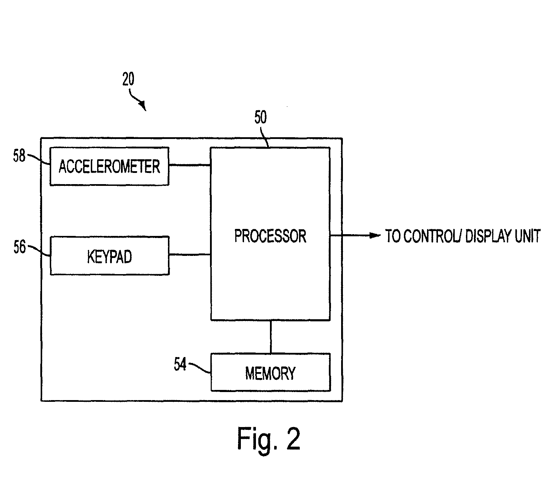

A user can press one of the inputs 32 on the keypad 56 to initiate movement based control of the distal end 14 of the probe 26. When initiated, movement based control can allow a user to move the operator control input unit 20 to control the distal end 14 of the probe 26. Movement based control can be initiated in a number of ways through the operator control input unit 20. In one example, the user can press one of the inputs 32 a first time to start the movement based control, and press one of the inputs 32 a second time to stop the movement based control. As such, when the user presses one of the inputs 32 the first time, the movement based control begins. To stop the movement based control, the user can press one of the inputs 32 for a second time. In another example, the user can press and hold one of the inputs 32 to start the movement based control, and can release one of the inputs 32 to stop the movement based control. Accordingly, the user can selectively choose when to activate and deactivate the movement based control by pressing and releasing one of the inputs 32, or by pressing and holding one of the inputs 32. However, the control via the inputs 32 may have some limitation, shortcoming or the like. As such, in accordance with an aspect of the present invention the operator control input unit 20 includes an accelerometer 58 (Fig. 2) for sensing movement of the operator control input unit imparted by the user (e.g., manipulation by the user) as a control input. The sensed movement is used to control movement of the video probe 26.

It is to be appreciated that Fig. 2 shows the components of the operator control input unit 20 as function blocks and that the function blocks are to represent any of a variety of structure that can perform the specified functions. The accelerometer 58 is operatively connected to a processor 50, which in turn is operatively connected to a memory 54 and the keypad 56. The accelerometer 58, processor 50, memory 54 etc. are housed within the operator control input unit 20. The processor 50 is operatively connected o the control/display unit 22 via the transmission line 30.

The accelerometer 58 can detect movement/acceleration of the operator control input unit 20 in multiple directions. Once the accelerometer 58 detects motion in the operator control input unit 20, the processor interprets the sensed movement and/or acceleration and transmits one or more corresponding control signals to the control/display unit 22, which in turn controls the motion of the video probe 26. The signals from the accelerometer 58 and processor 50 can be digital signals.

The accelerometer 58 can include a variety of different types of accelerometers to detect motion/acceleration. For instance, the accelerometer 58 could include a three-axis accelerometer. In such an example, the three-axis accelerometer could detect linear acceleration in three directions, including an up/down direction (Y-axis), left/right direction (X-axis), and forward/backward direction (Z-axis). In a further example, the accelerometer 58 could include a two-axis accelerometer. In such an example, the two-axis accelerometer could detect linear acceleration in two directions, including, but not limited to, the left/right direction (X-axis) and forward/backward direction (Z-axis). Furthermore, the accelerometer 58 could include a single-axis accelerometer, which detects linear acceleration in a single direction, such as, for example, the forward/backward direction (Z-axis). In further examples, the operator control input unit 20 could include one or more accelerometers. For instance, multiple-axis accelerometers could be assembled from a plurality of single-axis accelerometers oriented in different directions.

Motion detection of the example operator control input unit 20 and use of such for control of the video probe 26 can now be discussed. The user can press one of the inputs 32 to initiate the movement based control. For example, the use could press a certain one of the inputs 32 once, or press and hold a certain one of the inputs 32. The movement based control will then start, and the accelerometer 58 can detect the initial position of the operator control input unit 20 (e.g., 10° with respect to vertical). The accelerometer 58 conveys this initial position to the processor 50, which can store the initial position in the memory 54. Some or all subsequent motion of the operator control input unit 20 can be measured with respect to this initial position, and can be compared to a minimum and maximum threshold of motion stored by the memory 54. For instance, unintended tremors by the user will not rise above the minimum threshold, and will not be associated with command signals. Thus, the distal end 14 of the probe will not move. Similarly, large movements, such as dropping the operator control input unit 20, will rise above the maximum threshold, and will not be associated with command signals. Thus, the distal end 14 of the probe will not move.

Some or all of the subsequent movements of the operator control input unit 20 can be compared with respect to the initial position (e.g., 10° with respect to vertical). Accordingly, if the user moves the operator control input unit 20 in a leftward direction, then the accelerometer 58 can sense this movement to the left, and can send a signal to the processor 50 corresponding to leftward movement. Similarly, the accelerometer 58 can detect and send signals corresponding to nearly any movement of the operator control input unit 20, such as up, down, left, right, forward, backward, rotation, etc. The accelerometer 58 can continuously send motion signals to the processor 50 during the movement based control corresponding to movement of the operator control input unit 20.

The processor 50 receives the digital motion signals from the accelerometer 58 and/or inputs from the keypad 56 for operation thereon of information conveyed by such signals/inputs. The processor 50 can also send information and/or data to the memory 54 for storage. For instance, when the processor 50 receives the motion signal corresponding to the initial position of the operator control input unit 20, the processor 50 can store this initial position in the memory 54. The processor 50 can then compare any subsequent movements of the operator control input unit 20 with this initial position (e.g., 10° with respect to vertical). Thus, motion of the entire operator control input unit 20, which is very intuitive, rather that button actuation or the like, is used to control movement of the video probe 26.

It is contemplated that various additional features could be employed. For example, the processor 50 can selectively filter out some of the motion signals receive from the accelerometer 58 by excluding and/or disregarding some of the motion signals. These signals could include motion signals that correspond to involuntary movements by the user. For instance, it would be difficult for a user holding the operator control input unit 20 to remain perfectly still, and the user's hand will often experience unintended tremors. However, when the user holds the operator control input unit 20, the accelerometer 58 will detect unintended tremors in the user's hand, and transmit corresponding motion signals to the processor 50. Accordingly, the processor 50 can filter out and disregard any signals from the accelerometer 58 that fall below a certain, pre-set minimum threshold of motion.

The processor 50 can also filter out and disregard some or all signals from the accelerometer 58 that rise above a certain, pre-set maximum threshold of motion. For instance, if the user drops the operator control input unit 20, the accelerometer 58 can detect this unintended sudden change in direction and transmit corresponding motion signals to the processor 50. In this example, the processor 50 could filter out and disregard these sudden, sharp movements, which rise above the pre-set maximum threshold of motion. The minimum threshold and maximum threshold of motion can be stored in the memory 54, such that the processor 50 can compare any signals from the accelerometer 58 with the minimum and maximum thresholds that are stored in the memory 54.

The processor 50 can associate motion signals that are not filtered out with commands. Specifically, the processor can associate and/or correspond to the motion signals from the accelerometer 58 with commands for articulating the distal end 14 of the probe 26. If the operator control input unit 20 is moved or tilted to the left, the accelerometer 58 senses leftward motion/tilt and can transmit a motion signal to the processor 50. The processor 50 can then associate the leftward motion signal to a leftward command. The processor 50 can associate some or all of the signals from the operator control input unit 20 to commands. Similarly, the processor 50 can associate a motion signal indicating a larger movement, such as a sharp tilt or sweeping leftward movement of the operator control input unit 20, with a command to move the distal end 14 of the probe 26 a relatively larger distance.

It is to be understood that the processor 50 can produce a single command or multiple commands based on the motion signals. For instance, a single leftward movement of the operator control input unit 20 could produce a single motion signal and, thus, a single command. However, the user could continuously move the operator control input unit 20, such that the accelerometer 58 senses continuous motion and transmits one or more motion signals. In such an example, the processor 50 could produce multiple commands based on the motion signal(s) received from the accelerometer 58.

Referring now to Figs. 1 and 2, operation of the articulated video probe system 10 can now be described. A user can insert the distal end 14 of the probe 26 and the camera 12 into an area. The user may be looking for a specific structure and/or problem within the area. The camera 12 can display images and/or live streaming video of the area to the screen 29 of the control/display unit 22. The screen 29 can display real-time video of the area, as captured by the camera 12. In response to what the user sees on the screen 29, the user can move/articulate the probe 26 to move the camera 12, such that a different portion of the area can be displayed. To accomplish this, the user can initiate the movement based control of the operator control input unit 20.

The user can move the operator control input unit 20, causing the accelerometer 58 to detect and measure the acceleration and motion of the operator control input unit 20. For instance, the user can tilt the operator control input unit 20 in a variety of directions, or can move the operator control input unit 20 side to side, up and down, forward and backward, etc. In response to these movements, the accelerometer 58 can produce and transmit motion signals to the processor 50 that correspond with the movement of the operator control input unit 20. The processor 50 can convert these motion signals to commands, and can transmit these commands to the control/display unit 22 by the transmitter 52. Once the commands are received by the control/display unit 22, the control/display unit 22 can utilize the commands to move the distal end 14 of the probe 26 accordingly. For instance, a leftward tilt of the operator control input unit 20 could produce a leftward command, thus causing the control/display unit 22 to move the distal end 14 and, thus, the camera 12, in a leftward direction. The user can watch real time images from the camera 12 on the screen 29, and can adjust the position of the camera 12 by moving and/or tilting the operator control input unit 20.

It should be appreciated that the shown example has a connection between the operator control input unit 20 and the control/display unit 22 through the transmission line 30. However, it is contemplated that the connection may be via other means, such as a transmitter. The control/display unit 22 could then utilize the commands to articulate the probe 26 in response to transmitted commands from the operator control input unit 20. For example, if the operator control input unit 20 is tilted to the left, a leftward command is transmitted to the control/display unit 22 via the transmitter 52. The control/display unit 22 will receive this command, and utilize this command to articulate the probe 26. Specifically, the distal end 14 can be articulated in a leftward direction in accordance with the command from the operator control input unit 20.

Also, it should be appreciated that the shown example provides the operator control input unit 20 and the control/display unit 22 as separate such that the operator control input unit 20 is separately movable. However, it is contemplated that the operator control input unit 20 and the control/display unit 22 are integrated into a single unit.

The articulated video probe system 10 can be used in a variety of areas and applications. The articulated video probe system 10 can be used in areas that are inaccessible and/or hard to reach. The user may use the articulated video probe system 10 and insert the camera 12 into the area, such as through an opening. The camera 12 can deliver images to a location outside of the area, such as a video monitor, computer screen, display screen, television, or the like. Examples of areas that the camera 12 can be used include, but are not limited to, airplane components, such as engines, fuel tanks, wings, etc. Furthermore, the area could include non-airplane applications as well, including, but not limited to, cars, boats, tanks, pipes, furnaces, etc.

The articulated video probe system 10 described herein could include a number of different structures. For instance, the articulated video probe system 10 could be used as an improvement in accordance with a number of different VideoProbe® inspection systems. In one example, the articulated video probe system 10 could be used with an XL Go VideoProbe® inspection system. In another example, the articulated video probe system 10 could be used with an XLG3 VideoProbe® remote visual inspection system. In a further example, the articulated video probe system 10 could be used with an XL Vu VideoProbe® system. In yet another example, the articulated video probe system 10 could be used with a LongSteer® VideoProbe® inspection system. It is to be understood that further devices and structures, though not mentioned here, are also contemplated.

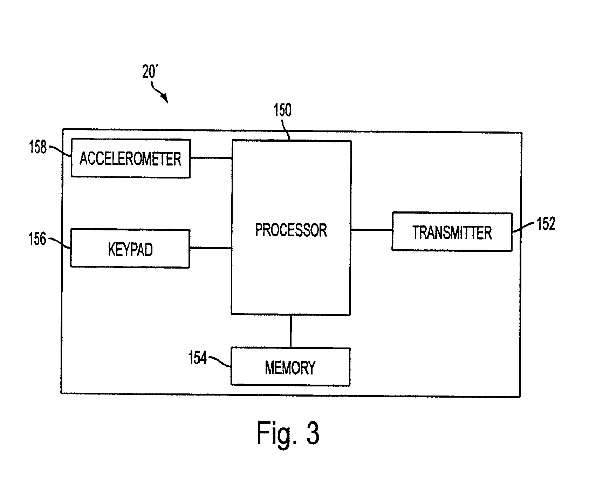

Referring now to Fig. 3, another example aspect in accordance with the present invention is shown. In this example, the operator control input unit 20' is integrated to and/or a shared arrangement with a variety of other components/devices. For instance, the operator control input unit 20' could include a number of handheld devices, including, but not limited to, a cell phone, a smart phone, a mobile device, an iDevice, a GPS style locator, etc. Accordingly, the user can use the handheld device, such as a cell phone, as the operator control input unit 20'. In these examples, some structures could be commonly shared by multiple functions/features of the integrated/shared arrangement, such as buttons, joysticks, accelerometer(s), or the like.

The operator control input unit 20', as integrated and/or be a shared arrangement with a variety of other components/devices, is freestanding from the control/display unit 22. As such, the operator control input unit 20' further includes a transmitter 152 for communication in lieu of the transmission line 30. A corresponding receiver would be employed within the associated control/display unit.

Other components of the operator control input unit 20' can be similar/identical to previously discussed components shown within Fig. 2. The transmitter 152 can be controlled by the processor 150 and can transmit signals and/or commands from the processor 150 to a control system for the articulation cables (not shown). The transmitter 152 can further transmit commands, thus causing the distal end 14 of the probe 26 to move as well.

Many of the mentioned handheld components/devices, including, but not limited to, a cell phone, a smart phone, a mobile device, an iDevice, a GPS style locator, etc. that could be within a shared arrangement may contain an accelerometer for other purposes. Thus, the physical structure of the accelerometer could be employed for an additional function of providing control input in accordance with an aspect of the present invention.

In a further example, the operator control input 20' can be included in the control/display unit 22. Specifically, the components of the operator control input 20', including the accelerometer 158, keypad 156, memory 154, transmitter 152, and/or processor 150, could be incorporated into the control/display unit 22. In such an example, a handheld component/device, such as the control input unit 20, may not be provided. Instead, the accelerometer 158 and other components of the control input 20' can be provided in the control/display unit 22. Accordingly, in this example, the video probe system 10 may include the control/display unit 22 as the single device that controls movement of the video probe 26. As such, in the example shown in FIG. 3, the control/display unit 22 can be moved, with the accelerometer 158 in the control/display unit 22 utilizing a received movement command signal to move the articulated video probe. The operation of the control/display unit 22 can be similar and/or identical to the operation of the control input unit 20, described above.

The invention has been described with reference to the example embodiments described above. Modifications and alterations will occur to others upon a reading and understanding of this specification. Example embodiments incorporating one or more aspects of the invention are intended to include all such modifications and alterations insofar as they come within the scope of the appended claims.

## Claims

1. An articulated video probe system (10) including:
an articulated video probe (26) for in-taking and transmitting an image of an area, the articulated video probe (26) being moveable for changing the in-taken image;
a unit (22) for causing the video probe (26) to move, the unit utilizing a received movement command signal to move the articulated video probe (26); and
an accelerometer (58) configured to sense motion and transmit the movement command signal to the unit (22) for causing the video probe (26) to move.

2. The articulated video probe system (10) of claim 1, wherein the video probe (26) includes a camera (12) configured to intake and transmit images of the area.

3. The articulated video probe system (10) of claim 1 or claim 2, including a display apparatus (24) for displaying images of the area transmitted by the video probe (26).

4. The articulated video probe system (10) of claim 1, 2 or 3, wherein the accelerometer (58) is located within a unit (20) that is manipulated by a user.

5. The articulated video probe system (10) of claim 4, wherein the accelerometer (58) is configured to sense an initial orientation of the unit (20) that is manipulated by a user.

6. The articulated video probe system (10) of claim 5, wherein the accelerometer (58) is configured to sense a change in motion of the unit (20) that is manipulated by a user with respect to the initial orientation of the unit (20) that is manipulated by a user.

7. A method of operating a video probe (26) system which has an articulated video probe (26) for in-taking and transmitting an image of an area, the method including:
sensing motion via an accelerometer (58) and transmitting a movement command signal indicative of the sensed motion;
utilizing the movement command signal at a unit (22) to cause the video probe (26) to move; and
moving the articulated video probe (26) for changing the in-taken image.

8. The method of claim 7, wherein the video probe (26) includes a camera (12) and the method includes in-taking and transmitting images of the area.

9. The method of claim 7 or claim 8, wherein the video probe (26) include a display apparatus (24) and the method includes displaying images of the area transmitted by the video probe (26) upon the display apparatus (24).

10. The method of claim 7, 8 or 9, wherein the accelerometer (58) is located within a unit (20) that is manipulatable by a user, and the method includes the user manipulating the unit (20).

11. The method of claim 10, wherein the accelerometer (58) senses an initial orientation of the unit (20) that is manipulated by a user.

12. The method of claim 11, wherein the accelerometer (58) senses a change in motion of the unit (20) that is manipulated by a user with respect to the initial orientation of the unit (20) that is manipulated by a user.
